(19) Europäisches Patentamt
European Patent Office
Office européen des brevets

(11) Numéro de publication : **0 461 972 B1**

(12) **FASCICULE DE BREVET EUROPEEN**

(45) Date de publication du fascicule du brevet : **30.03.94 Bulletin 94/13**

(51) Int. Cl.$^5$ : **C07C 45/51,** C07C 45/56, C07C 47/21, C07C 47/42, C07C 47/54, C07C 209/60, C07C 291/04

(21) Numéro de dépôt : **91401508.6**

(22) Date de dépôt : **10.06.91**

(54) **Procédé de transformation de N-oxydes d'amines tertiaires en aldéhydes.**

(30) Priorité : **11.06.90 FR 9007224**

(43) Date de publication de la demande : **18.12.91 Bulletin 91/51**

(45) Mention de la délivrance du brevet : **30.03.94 Bulletin 94/13**

(84) Etats contractants désignés : **AT BE CH DE DK ES FR GB GR IT LI LU NL SE**

(56) Documents cités :
**EP-A- 0 051 229**
**FR-A- 2 320 280**
**GB-A- 2 032 422**
**US-A- 4 533 751**
**JACS, vol. 83, 1961, pages 1871-1878; J.C. CRAIG et al.: "Tertiary amine oxide rearrangements. I Mechanism"**
**J. ORG. CHEM., vol. 33, no. 9, 1968, pages 3493-3498; J.P. FERRIS et al.:"Detoxication mechanisms. III. The scope and mechanism of the iron-catalyzed dealkylation of tertiary amine oxides"**

(56) Documents cités :
**SYNTHESIS, 1985, pages 770-773; I. MONKOVIC et al."Secondary amines from theiron(II) ion-catalyzed reaction of amine oxides: A general method for the dealkylation of tertiary amines"**

(73) Titulaire : **RHONE-POULENC NUTRITION ANIMALE**
**Rue Marcel Lingot**
**F-03600 Commentry (FR)**

(72) Inventeur : **Chabardes, Pierre**
**24 rue Jeanne d'Arc**
**F-69110 Sainte Foy les Lyon (FR)**
Inventeur : **Henrot, Serge**
**68 Mail des Basses Barrolles**
**F-69230 Saint Genis Laval (FR)**
Inventeur : **Mercier, Claude**
**85 avenue du Point du Jour**
**F-69005 Lyon (FR)**

(74) Mandataire : **Savina, Jacques et al**
**RHONE-POULENC RORER S.A., Direction des Brevets, 20 avenue Raymond Aron**
**F-92160 Antony Cédex (FR)**

EP 0 461 972 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention concerne le domaine des transformations de N-oxydes en aldéhydes, et plus particulièrement des transformations de N-oxydes d'amines tertiaires en aldéhydes. Elle concerne également le domaine des synthèses d'aldéhydes.

Il est connu de convertir des N-oxydes d'amines tertiaires contenant au moins un groupement méthyl, en amines secondaires et en formaldéhyde en présence d'un excès d'anhydride acétique (réaction de Polonowsky).

Il est également connu d'utiliser cette réaction en synthèse terpénique (Takabe et Coll., Synthetic Communications, 13(4) 297-301, (1983)) et, plus précisément, pour la préparation du citral à partir du N-oxyde de la N-dialkylgéranylamine (brevet US 4 447 649).

Cependant, ce type de réaction consomme de l'anhydride acétique et, de plus, ne permet pas le recyclage de l'amine secondaire. Ce double inconvénient constitue un obstacle important à l'exploitation au niveau industriel.

Pour tenter de remédier à ces inconvénients, il a été proposé d'utiliser non plus l'anhydride acétique mais d'autres agents de transformation tels qu'en particulier $MnO_2$ (Henbest et Coll, Amine Oxydation part I, 3035, (1957)), $SO_2$ (Lecher et Coll, J.Am.Chem.Soc. 70, 3789, (1948)) ou différents sels métalliques.

On peut citer en particulier les travaux de Fish et Coll, (J.Am.Chem.Soc., 78, 3668, (1956)) sur les réarrangements de N-oxydes en présence d'ions ferriques.

Enfin, Ferris et Coll. (J.Org.Chem. 33, 3493, (1968)) et Craig et Coll. (J.Am.Chem.Soc. 83, 1871, (1961)) ont montré qu'il était possible d'utiliser d'autres métaux que le fer, et notamment des métaux de transition tels que le ruthénium, l'osmium et le vanadium. Toutefois, concernant ce dernier, seule son activité sur la transformation du N-oxyde de la triméthylamine a été exemplifiée. De plus, cette réaction est conduite uniquement en milieu aqueux, très dilué, en présence de quantités très élevées de métal de transition, $VOCl_2$, (trois fois plus que de N-oxyde, ce qui ne peut être considéré comme de la catalyse) et conduit de surcroit à la formation d'acide formique. Dans ces conditions, outre le rendement moyen, l'énorme consommation de dérivé métallique, l'acidité très élevée du milieu réactionnel (pH 1,5) et la grande dilution font qu'il n'est pas possible de transposer aisément ce procédé à l'échelle industrielle.

Il a maintenant été trouvé, et c'est ce qui fait l'objet de la présente invention, qu'il est possible de transformer les N-oxydes d'amines tertiaires en aldéhydes et en amines secondaires avec des rendements élevés, en opérant en présence d'une quantité catalytique d'un ou plusieurs dérivés du vanadium. De plus, le procédé de l'invention présente l'avantage de ne pas former d'acide et de permettre de récupérer facilement l'amine secondaire. Enfin, il est possible de mettre en oeuvre ce procédé dans des conditions de pH et de température très avantageuses.

Un objet de la présente invention concerne donc un procédé de préparation d'aldéhydes dans lequel on fait réagir un N-oxyde d'amine tertiaire dans un milieu aqueux en présence d'un catalyseur à base de vanadium caractérisé en ce que l'on opère en présence d'un solvant organique et d'une quantité catalytique d'un ou de plusieurs dérivés du vanadium.

Selon la présente invention, le terme "quantité catalytique" signifie que le rapport molaire dérivé du vanadium (compté en métal) / N-oxyde est inférieur à 1.

Les N-oxydes d'amines tertiaires utilisés dans la présente invention peuvent être obtenus à partir de l'amine correspondante, par oxydation en présence d'un agent oxydant. On peut en particulier utiliser l'eau oxygénée, seule ou sous pression de gaz carbonique, selon le procédé décrit dans le brevet US 4 247 480.

Les N-oxydes d'amines tertiaires utilisables dans le présente invention répondent notamment à la formule générale (I) :

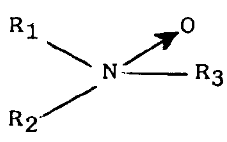

(I)

dans laquelle $R_1$, $R_2$ et $R_3$, égaux ou différents, sont choisis parmi les groupements alkyl ou alkényl, linéaires, ramifiés ou cycliques, ou les groupements benzyl, lesquels groupements, substitués ou non, contiennent 1 à 30 atomes de carbone et éventuellement un ou plusieurs hétéroatomes, et dans laquelle $R_1$ et $R_2$ peuvent former entre-eux un cycle.

Avantageusement, $R_1$ et $R_2$, égaux ou différents, sont des groupements alkyl, linéaires ou ramifiés, contenant 1 à 4 atomes de carbone et pouvant former entre-eux un cycle ou un hétérocycle.

Encore plus avantageusement, $R_1$ et $R_2$, égaux ou différents, représentent un groupement méthyl ou éthyl.

Selon une première préférence, dans la formule (I), $R_3$ est un groupement allylique ayant entre 3 et 30 atomes de carbone, linéaire ou ramifié, substitué ou non, contenant ou non un cycle, et contenant éventuellement un ou plusieurs hétéroatomes.

Selon une seconde préférence, dans la formule (I), $R_3$ est un groupement benzylique substitué ou non contenant jusqu'à 30 atomes de carbone. En particulier, le noyau benzylique peut être substitué par des radicaux alkyle, alcényle, acyle, alkoxy, hydroxyle, ou par des radicaux de formule CHO, -R4-COOR5, ou -R4-X dans lesquelles R4 représente un lien valentiel ou un radical hydrocarboné divalent, R5 représente un atome d'hydrogène ou un groupement alkyle, et X symbolise un atome d'halogène.

Préférentiellement, $R_3$ est soit un groupement allylique contenant entre 3 et 15 atomes de carbone, soit un groupement benzylique dont le noyau aromatique peut être substitué.

Encore plus préférentiellement, les N-oxydes de formule (I) peuvent être choisis parmi le N-oxyde de la N-dialkylprénylamine, le N-oxyde de la N-dialkylgéranylamine ou de son isomère la N-dialkylnérylamine, le N-oxyde de la N-dialkylbenzylamine ou le N-oxyde de la N-dialkyl(triméthyl-2,6,6 cyclohéxène-1) méthanamine.

Dans ce qui suit, nous entendons sous le terme géranylamine le mélange des isomères géranyl et nérylamines.

Les dérivés du vanadium utilisables notamment dans le procédé selon l'invention sont des sels de vanadium à tous les degrés d'oxydation possibles. Dans les degrés d'oxydation élevés, on peut citer en particulier $V_2O_5$, $NH_4VO_3$, les vanadate d'alcoyle tels que l'orthovanadate de triéthanolamine (OVTEA) et l'orthovanadate d'octadécyle ($OV(OC_{18}H_{37})_3$) (pour le degré 5), l'acétylacétonate de vanadyle ($OV(AcAc)_{2)}$ ou le sulfate de vanadyle ($OVSO_4$) pour le degré 4.

Par ailleurs, ces dérivés peuvent être utilisés en suspension ou supportés. Dans ce dernier cas, on peut utiliser les supports de type oxyde, tels que notamment les alumines ou les silices. On peut également utiliser les charbons ou encore les résines.

Dans un mode préféré de mise en oeuvre du procédé selon l'invention, la quantité de catalyseur au vanadium est telle que le rapport molaire dérivés du vanadium (compté en métal) / N-oxyde est compris entre 0,0001 et 0,5, et de préférence, entre 0,001 et 0,1.

En ce qui concerne le solvant, le procédé peut être mis en oeuvre en utilisant un hydrocarbure, un éther, un ester, un alcool, un solvant aromatique ou un solvant halogéné. On peut mentionner comme hydrocarbure le pentane ou l'hexane, comme ester l'acétate d'éthyle et comme éther le méthylterbutyléther, comme solvants halogénés le chlorure de méthylène ou le chlorobenzène, comme solvants aromatiques le toluène le xylène, le benzène ou comme solvants alcooliques le méthanol, cette liste n'étant bien sûr pas exhaustive.

Le pH du milieu réactionnel est avantageusement ajusté par l'ajout d'un acide organique ou minéral, ou des deux. On peut en particulier utiliser des acides tels que l'acide acétique, l'acide benzoïque, l'acide tartrique, l'acide formique ou l'acide crotonique, seuls ou avec addition à pH contrôlé d'un acide organique ou minéral tels que l'acide chlorhydrique ou l'acide sulfurique. Avantageusement, on utilise un acide carboxylique. Le pH du milieu est ajusté de préférence à une valeur comprise entre 4 et 7.

Dans un mode préféré de mise en oeuvre de l'invention, le milieu réactionnel contient, par mole de N-oxyde, entre 0,05 et 2 litres d'eau, et un volume de solvant compris entre 0 et 20 litres et de préférence entre 0,5 et 10 litres.

Avantageusement, le procédé de l'invention est conduit à une température comprise entre la température ambiante et 150°C, éventuellement sous la pression autogène des réactifs.

Le procédé de l'invention permet de plus de recycler l'amine secondaire générée par la réaction de transformation, avec un très bon rendement. Dans une variante particulièrement avantageuse, le procédé de l'invention consiste donc à faire réagir le N-oxyde d'amine tertiaire sur le ou les dérivés du vanadium, puis à récupérer, dans la phase organique l'aldéhyde, et dans la phase aqueuse l'amine secondaire.

Par ailleurs, la demanderesse a montré que le procédé décrit ci-avant permettait de préparer des aldéhydes insaturés directement à partir de diènes conjugués et d'amines secondaires, sans isoler les intermédiaires réactionnels.

Un autre objet de l'invention réside donc dans un procédé de préparation d'aldéhydes insaturés caractérisé en ce que l'on effectue, sans isoler les intermédiaires réactionnels, les étapes suivantes:

. dans une première étape, on condense un diène conjugué et une amine secondaire,
. dans une deuxième étape, on oxyde l'amine tertiaire ainsi obtenue au moyen d'un agent oxydant, et
. dans une troisième étape, on transforme le N-oxyde d'amine tertiaire obtenu en aldéhyde insaturée en utilisant le procédé décrit ci-avant.

Cet objet de l'invention fournit un procédé amélioré de synthèse d'aldéhydes, particulièrement adapté et performant pour une exploitation industrielle.

Les diènes conjugués utilisables dans l'invention peuvent être choisis parmi les groupements alkényl linéaires, ramifiés, ou cycliques, substitués ou non, contenant 1 à 30 atomes de carbone et éventuellement 1 ou plusieurs hétéroatomes.

Avantageusement, les diènes conjugués utilisables dans l'invention répondent à la même formule que les groupements R3 précédemment définis.

A titre d'exemple de diènes conjugués utilisables dans le procédé de l'invention, on peut citer le myrcène, l'isoprène, le butadiène, le farnésène ou le phytadiène.

On peut utiliser dans la présente invention les amines secondaires de formule HNR1R2 dans laquelle R1 et R2, égaux ou différents sont définis comme précédemment.

A titre d'exemple on peut citer la diéthylamine qui donne de très bons résultats.

La première étape du procédé de l'invention peut être réalisée de plusieurs manières. Généralement, on préfère utiliser un catalyseur métallique. Notamment, les métaux utilisables dans cette étape de l'invention peuvent être des métaux alcalins. On peut citer en particulier le sodium, le lithium ou le potassium métalliques. Plus généralement, les catalyseurs décrits par Marata et coll (Nippon Kagaku Kaishi, 1233, 1982) conviennent dans le procédé de l'invention.

Dans un mode particulier de mise en oeuvre, il est possible d'opérer en présence d'un agent activateur, pour augmenter le rendement ou la vitesse de la réaction. En particulier, il est possible d'ajouter au métal alcalin du biphényle ou du naphtalène.

Dans un mode préféré de mise en oeuvre de l'invention, le rapport molaire amine/diène est compris entre 0,1 et 5, et de préférence entre 0,5 et 2.

Avantageusement, on préfère opérer en présence d'un léger excès d'amine.

La réaction est avantageusement conduite à une température comprise entre la température ambiante et la température de reflux. Il est entendu que les conditions réactionnelles (température, temps de réaction, rapport des réactifs ..) sont adaptées par l'homme de l'art en fonction de la vitesse ou du rendement recherchés.

Préférentiellement, le rapport molaire catalyseur/diène est compris entre 0,001 et 0,1.

Avant d'engager la deuxième étape du procédé de l'invention, il peut être préférable de transformer le métal alcalin en hydroxyde ou alcoxy correspondant par ajout d'eau ou d'alcool dans le milieu alcalin.

La deuxième étape consiste à former le N-oxyde de l'amine tertiaire au moyen d'un agent oxydant.

Préférentiellement, on utilise comme agent oxydant l'eau oxygénée, éventuellement sous atmosphère ou pression de $CO_2$. Avantageusement, la quantité d'eau oxygénée est telle que le rapport molaire eau oxygénée / diène conjugué est proche de la stoechiométrie. Encore plus avantageusement, on préfère utiliser un léger excès d'eau oxygénée par rapport à l'amine tertiaire formée.

La réaction d'oxydation peut éventuellement être conduite en présence d'un solvant, selon la nature du diène conjugué et de l'amine utilisés. Notamment, il est possible d'effectuer l'oxydation en milieu alcoolique (méthanol), acide organique (acide acétique), ou présence d'acétone.

Avantageusement, on utilise un solvant compatible avec la dernière étape du procédé.

Les autres paramètres de la réaction (température, temps ..) sont adaptées par l'homme de l'art en fonction du diène et de l'amine utilisés et de la vitesse de réaction recherchée. La température de la réaction est avantageusement comprise entre la température ambiante et 100°C.

La troisième étape consiste à transformer le N-oxyde d'amine tertiaire formé lors de l'étape précédente en aldéhyde insaturé, en utilisant un ou plusieurs dérivés du vanadium en quantité catalytique, selon le procédé décrit ci-avant.

Ce procédé de synthèse d'aldéhydes apporte au niveau industriel de nombreux avantages. En particulier, le procédé de l'invention est simple dans sa mise en oeuvre (limitation du nombre d'appareils utilisés, affranchissement de la manipulation d'intermédiaires ..) ce qui se traduit par un gain de temps et par un gain économique. Par ailleurs, le procédé de l'invention permet de régénérer avec un très bon rendement l'amine secondaire de départ, qui peut de nouveau réagir sur un diène conjugué. Encore selon l'invention, les réactions secondaires sur les intermédiaires réactionnels (tel que notamment le réarrangement du N-oxyde selon le mécanisme décrit par Meisenheimer, Ber.52, 1667 (1919)) sont diminuées, ce qui améliore les rendements du procédé.

La présente invention sera plus complètement décrite à l'aide des exemples suivants, qui doivent être considérés comme illustratifs et non limitatifs.

EXEMPLE 1

Dans un ballon tricol de 1 litre, muni d'une agitation, d'un réfrigérant, d'une ampoule de coulée et d'un thermomètre, on charge :

- 0,2 g de $VOSO_4$ à 22,75% en vanadium
- 80 ml d'une solution aqueuse à 10% d'acide acétique

- 400 ml de chlorure de méthylène

On agite et porte au reflux du chlorure de méthylène (38°C). On coule alors une solution aqueuse de 13,6 g de N-oxyde de la N-diéthylgéranylamine à 58%, dissout dans 40 ml de chlorure de méthylène (durée d'addition 11 minutes), puis rince l'ampoule avec 20 ml de chlorure de méthylène. Le pH est de 4,1.

On chauffe 1 heure à 38°C, et refroidit rapidement à température ambiante. On décante la phase organique que l'on filtre rapidement sur 250 g d'alumine d'activité 4.

Le dosage du citral dans cette phase organique par chromatographie en phase vapeur donne un rendement de 75% par rapport au N-oxyde.

## EXEMPLE 2

Cet exemple a été réalisé dans les conditions décrites par Craig et Coll. Indépendemment des problèmes de mise en oeuvre, il illustre la faible efficacité de ce procédé.

Dans un ballon tricol de 3 litres, muni d'une agitation, d'un réfrigérant, d'une ampoule de coulée et d'un thermomètre, on charge :

- 2,04 litres d'eau
- 7,59 g de $VOSO_4$, $5H_2O$

On agite et chauffe à 80°C, puis ajoute,

- 3,95 g d'une solution de N-oxyde de la N-diéthyl géranylamine à 58% utilisée dans l'exemple précedent.

Le pH est de 2,4. On chauffe 40 minutes à 80°C, puis refroidit, et extrait par 3x100 ml de chlorure de méthylène. Le dosage du citral dans la phase organique comme dans l'exemple 1 donne un rendement en citral de 2%. On ne met pas en evidence d'acide géranique.

## EXEMPLE 3

Cet exemple illustre les possibilités de récupération de l'amine secondaire, lorsque l'on utilise le procédé de l'invention.

Dans un ballon tricol de 1 litre, muni d'une agitation, d'un réfrigérant, d'une ampoule de coulée et d'un thermomètre, on charge :

- 13,8 g de N-oxyde de la N-diéthylgéranylamine à 67,7% en solution aqueuse,
- 0,2 g de $OVSO_4$, $5H_20$,
- 50 ml d'une solution aqueuse à 10% d'acide acétique,
- 100 ml de pentane.

On chauffe à reflux (33°C) pendant 16 heures. Après refroidissement puis décantation, on extrait la couche aqueuse par 2x50 ml de pentane. Après concentration, on obtient 5,5g d'un brut dans lequel on dose le citral. Le rendement est de 48,8%. La distillation de ce brut donne 2,8 g de citral (rendement 45%).

La couche aqueuse est amenée à pH 10 par addition de soude, puis chauffée à 100°C en distillant, ce qui permet de récupérer 1,9 g de diéthylamine, soit un rendement de 64% par rapport au N-oxyde engagé.

## EXEMPLE 4

Dans un réacteur, on charge 7,5 ml d'acide acétique à 10 %, 3,39 g du N-oxyde de la diéthylgéranylamine, 25 ml de pentane et un étalon interne qui permettra de savoir à tout moment le rendement en aldéhyde formé. On dissout 0,05 g de $VOSO_4$, $5H_2O$ dans 5 ml d'acide acétique à 10 % que l'on coule en 25 minutes dans le réacteur, tout en maintenant la température à 35°C. On suit ensuite la formation du citral en fonction du temps par dosage en chromatographie en phase gazeuse.

RR = Rendement Réel = nombre de moles formées sur le nombre de moles engagées.

| Temps (min) | 35 | 75 | 120 | 240 | 300 | 360 | 420 | 450 | 480 |
|---|---|---|---|---|---|---|---|---|---|
| RR (%) | 1,6 | 8 | 14,3 | 31,9 | 41,9 | 47,4 | 52,6 | 54,3 | 55,3 |

EXEMPLE 5

On opère dans un ballon de 50 ml, muni d'un réfrigérant, d'un thermomètre et d'une agitation et possédant un septum pour introduire le N-oxyde.

On charge $4.10^{-5}$ mole de $OVSO_4$ (comptées en métal) et un étalon interne (carbure en C11) qui permettra de savoir à tout moment le rendement en aldéhyde. On introduit ensuite 4 ml d'acide acétique à 10 %, 20 ml de chlorobenzène et la température est ajustée à 60°C. $2.10^{-3}$ moles du N-oxyde de la N-diéthylgéranylamine diluées dans 3 ml de chlorobenzène sont alors introduites dans le ballon et le rendement réel en citral est mesuré par chromatographie en phase vapeur. Le pH de la réaction est compris entre 4 et 4,5.

Le rendement en citral est de 46% en 45 minutes.

EXEMPLE 6

On procède comme dans l'exemple 5, mais en présence de $NH_4VO_3$ au lieu de $OVSO_4$. Le rendement en citral est de 45% au bout de 90 minutes.

EXEMPLE 7

On procède comme dans l'exemple 5, mais en présence d'acétylacétonate de vanadyle au lieu de $OVSO_4$. Le rendement en citral obtenu est de 48% au bout de 18 minutes.

EXEMPLE 8

On opère comme dans l'exemple 5, mais en présence d'orthovanadate d'octadécyle au lieu de $OVSO_4$. Le rendement en citral obtenu est de 42% en 45 minutes.

EXEMPLE 9

On opère comme dans l'exemple 5, mais en présence d'orthovanadate de triéthanolamine à la place de $OVSO_4$. Le rendement en citral obtenu est de 32% en 90 minutes.

EXEMPLE 10

On opère comme dans l'exemple 5, mais en présence de $V_2O_5$ à la place de $OVSO_4$. Le rendement en citral obtenu est de 33% en 220 minutes.

EXEMPLE 11

On opère comme dans l'exemple 5, mais en présence de $4.10^{-6}$, $4.10^{-5}$ (exemple 2) ou $4.10^{-4}$ moles de $OVSO_4$ (comptées en métal). Les rendements en citral mesurés sont les suivants :

| Temps | | RR (%) | |
|---|---|---|---|
| (min) | $4.10^{-6}$ | $4.10^{-5}$ | $4.10^{-4}$ |
| 15 | | | 45 |
| 30 | 2,5 | 45 | |
| 345 | 32 | | |

On constate une diminution de la vitesse de la réaction avec la diminution de la quantité de catalyseur, mais le rendement réel en citral n'est pas affecté.

EXEMPLE 12

On opère comme dans l'exemple 5, mais en présence de chlorure de méthylène à la place du chlorobenzène et à une température de 37°C. Dans ces conditions, le rendement en citral obtenu est de 61% au bout de 55 minutes.

EXEMPLE 13

On opère comme dans l'exemple 5, mais en présence d'un mélange hexane-chlorobenzène 10/1 (rapport des volumes) comme solvant, à une température de 45°C environ. Dans ces conditions, le rendement en citral est de 45% au bout de 4 heures.

EXEMPLE 14

On opère comme dans l'exemple 5, mais en présence d'un mélange pentane-chlorure de méthylène 10/1 (rapport des volumes), à une température de 30°C pendant 21 heures. Dans ces conditions, le rendement en citral est de 61%.

EXEMPLE 15

On opère comme dans l'exemple 5, mais en présence de méthanol, d'acétate d'éthyle ou de toluène, à une température de 60°C environ. Les rendements obtenus sont les suivants :
- dans le méthanol : 18% de citral en 1 heure
- dans le toluène : 43% de citral en 1 heure
- dans l'AcOEt : 38% de citral en 4 heures

EXEMPLE 16

On procède comme dans l'exemple 5, mais en présence de terbutylméthyléther à 50°C. Le rendement obtenu est de 43% en 2 heures.

EXEMPLE 17

On procède comme dans l'exemple 5 mais en présence d'acide sulfurique. Le pH du milieu réactionnel est ajusté à une valeur comprise entre 4,5 et 5. Le rendement obtenu est de 32% en citral au bout de 3 heures.

EXEMPLE 18

On procède comme dans l'exemple 5. On charge parallèlement les trois solutions suivantes :
- 2,025g d'une solution aqueuse de N-oxyde de la N-diéthylgéranylamine à 67%
- 30,8 mg de $VOSO_4$ (à 22,75% en vanadium)
- 2,57 g d'acide benzoïque
- 12 ml d'eau
- 69 ml de chlorure de méthylène
- 314 mg de undecane (étalon interne)

Le pH s'établit aux alentours de 5

Rendement en citral après une heure à 38°C : 63%
- 0,682g d'une solution aqueuse de N-oxyde de la N-diéthylgéranylamine à 67%
- 10,6 mg de $VOSO_4$ (à 22,75% en vanadium)
- 0,322 g d'acide formique
- 4 ml d'eau
- 23 ml de chlorure de méthylène
- 102 mg de undecane (étalon interne)

Le pH s'établit aux alentours de 2,1 - 3.

Rendement en citral après 22 heures à 38°C : 62%
- 0,691g d'une solution aqueuse de N-oxyde de la N-diéthylgéranylamine à 67%
- 10,7 mg de $VOSO_4$ (à 22,75% en vanadium)
- 0,661 mg d'acide tartrique
- 4 ml d'eau
- 33 ml de chlorure de méthylène
- 118 mg de undecane (étalon interne)

Le pH s'établit aux alentours de 2,4.

Rendement en citral après 4 heures à 38°C : 61%

EXEMPLE 19

On procède comme dans l'exemple 5 mais en présence d'un acide organique et d'un acide minéral. Pour cela, l'appareillage est muni d'un dispositif pour injecter le second acide : solution 0,5M HCl à un débit de 0,7 ml/h. On charge :

- 0,687 g de la solution aqueuse de N-oxyde de la N-diéthylgéranylamine à 67%,
- 10,5 mg de $VOSO_4$,
- 0,12 g d'acide acétique
- 4 ml d'eau
- 23 ml de chlorure de méthylène.

On chauffe à 38°C et on injecte en 90 minutes 2,1 ml de la solution 0,5M d'HCl. Le pH se maintient entre 4,2 et 4,5.

Au bout de 4h40, le rendement en citral est de 60%.

EXEMPLE 20

On procède comme dans l'exemple 19. On charge :

- 0,675 g de la solution aqueuse de N-oxyde de la N-diéthylgéranylamine à 67%,
- 10,5 mg de $VOSO_4$,
- 87,6 mg d'acide crotonique
- 4 ml d'eau
- 23 ml de chlorure de méthylène.

On chauffe à 38°C et on injecte en 180 minutes 2,3 ml de la solution 0,5M d'HCl. Le pH se maintient entre 4,5 et 5.

Au bout de 3h30, le rendement en citral est de 51%

EXEMPLE 21

On opère comme dans l'exemple 5, mais en utilisant comme solvant un mélange pentane-chlorure de méthylène 10/1 (rapport de volumes) à une température de 30°C, et comme catalyseur, $NH_4VO_3$. On obtient un rendement de 50% en citral, au bout de 90 minutes.

EXEMPLE 22

Cet exemple illustre la possibilité d'utiliser des catalyseurs dérivé du vanadium supportés.

On procède comme dans l'exemple 5. On charge :

- 0,68 g de la solution aqueuse de N-oxyde de la N-diéthylgéranylamine à 67%,
- 117 mg de $V_2O_5$ à 3% sur $SiO_2$,
- 0,426 g d'acide acétique
- 4 ml d'eau
- 23 ml de chlorure de méthylène.

On chauffe à 37°C. Le rendement en citral est de 60% au bout de 3 heures.

EXEMPLE 23

On procède comme dans l'exemple 22. On charge :

- 0,68 g de la solution aqueuse de N-oxyde de la N-diéthylgéranylamine à 67%,
- 36,9 mg de $V_2O_5$ à 10% sur Alumine,
- 0,432 g d'acide acétique
- 4 ml d'eau
- 23 ml de chlorure de méthylène.

On chauffe à 37°C. Le rendement en citral est de 60% au bout de 3 heures.

EXEMPLE 24

On procède comme dans l'exemple 5. On charge :

- 2,67 g d'une solution aqueuse contenant 1,51 g de N-oxyde de la N-diméthylbenzylamine,
- 25 ml de chlorobenzène,
- 12,5 ml d'eau à 10% d'acide acétique,
- 0,5 g de undécane, et
- 0,05g de sulfate de vanadyle (à 22,75% de vanadium).

On chauffe à reflux à 89°C et on suit par chromatographie. Au bout de 15 minutes, le rendement en benzaldéhyde présent dans la couche organique est de 63%.

On arrête la réaction au bout de 135 minutes, et, après extraction de la couche aqueuse par 2x15 ml de chlorobenzène, on obtient le benzaldéhyde avec un rendement de 69%.

EXEMPLE 25

On procède comme dans l'exemple 5. On charge :

- 2,7 g d'une solution aqueuse contenant 1,33 g de N-oxyde de la N-diéthylprénylamine,
- 4,8 ml d'eau,
- 1,2 ml d'acide acétique, et
- 49 mg de sulfate de vanadyle (à 22,75% de vanadium).

On chauffe 4 heures au reflux à 62°C. Après refroidissement et décantation, on extrait par 2x25 ml d'hexane en saturant la couche aqueuse par NaCl.

Le dosage du prénal dans les phases hexaniques réunies, par chromatographie en phase vapeur donne un rendement de 65% par rapport au N-oxyde.

EXEMPLE 26

Dans un réacteur, on charge dans l'ordre suivant :

- 4,4 g d'une solution aqueuse contenant 15 mmoles de N-oxyde de la N-diméthylorthohydroxybenzylamine,
- 0,002 eq de $VOSO_4$
- 1,1 eq d'acide acétique, et
- 20 ml d'acétate d'éthyle.

On chauffe 5 heures à 60°C, et, après refroidissement, l'aldéhyde formé est dosé par CPV. On obtient un rendement réel de 26%.

EXEMPLE 27

Dans un réacteur de 50 ml équipé d'un réfrigérant, d'un thermomètre, d'une électrode de pH mètre et d'une agitation, on charge:

| | |
|---|---|
| undécane (étalon interne) | 500 mg |
| NH4VO3 | 23,5 mg |
| acide acétique | 0,3 g |
| eau | 4 ml |
| hexane | 20 ml |

On chauffe à 60°C et on ajoute 3,38 g du N-oxyde de la N-diéthylgéranylamine et 5 ml d'hexane. Au bout de 6 min., on commence à introduire à l'aide d'un pousse seringue de l'acide sulfurique 1N (débit 1,7 ml/h) de façon à maintenir le pH aux alentours de 4,5. Après 2h40, on a introduit un volume de 4,6 ml d'acide sulfurique 1N et le rendement en citral est de 45%.

EXEMPLE 28

On opère comme dans l'exemple 26. On charge :

| | |
|---|---|
| undécane | 112 mg |
| OVSO4 | 10,5 mg |
| acide crotonique | 87,6 mg |
| eau | 4 ml |
| chlorure de méthylène | 20 ml |

On chauffe à 38°C et on ajoute 675 mg du N-oxyde de la N-diéthylgéranylamine et 3 ml de chlorure de méthylène. Au bout de 2 min., on commence à introduire à l'aide d'un pousse seringue de l'acide chlorhydrique 0,5 M (débit 1,7 ml/h) de façon à maintenir le pH aux alentours de 4,5. Après 3h30, on a introduit un volume de 2,3 ml d'acide sulfurique 1N et le rendement en citral est de 51%.

EXEMPLE 29

Dans un tricol de 50 ml équipé d'un réfrigérant, d'un thermomètre, d'une électrode de pH mètre et d'une agitation, on charge:

| | |
|---|---|
| undécane (étalon interne) | 506 mg |
| NH4VO3 | 12 mg |
| acide acétique | 1,2 g |
| eau | 4 ml |
| hexane | 20 ml |

On chauffe à 60°C et on ajoute 3,37 g du N-oxyde de la N-diéthylgéranylamine et 5 ml d'hexane. On chauffe ensuite pendant 1 heure.

Après refroidissement, on prélève la couche organique qui est évaporée et analysée pour connaitre sa teneur en citral.

Sur la phase aqueuse on charge:

undécane, N-oxyde, héxane (mêmes quantités que précédemment) et un appoint d'acide acétique (0,6 g). On chauffe ensuite pendant 1 h, puis opère comme précédemment. On effectue ainsi, sur le même pied catalytique, 9 transformations. Les rendements en citral sont donnés dans le tableau suivant:

| Charge | Durée(h) | Rendement citral |
|---|---|---|
| 1 | 1 | 53.6 |
| 2 | 1 | 47.2 |
| 3 | 1,30 | 43.7 |
| 4 | 1 | 46.1 |
| 5 | 1 | 46.7 |
| 6 | 1 | 46.1 |
| 7 | 1,45 | 46 |
| 8 | 2,15 | 44.6 |
| 9 | 1,10 | 47 |

EXEMPLE 30

Dans un ballon, on place 0,2g (0,03 mole) de lithium métal et 20% de la quantité totale (1,23 moles) de diéthylamine. Le réacteur est chauffé une heure à reflux. On coule le mélange composé de myrcène à 95% 143g (1 mole) et le reste de la diéthylamine 72,4 g en 1 heure. La réaction est laissée 5 heures à reflux. Après refroidissement, on ajoute 5g d'eau. L'excès de diéthylamine, le myrcène non réagi et l'eau sont éliminés par distillation (à 70°C sous 27 mm de mercure). On obtient un culot de 176,7 g, qui est placé sous atmosphère de $CO_2$. On ajoute à 60°C et en 2 heures 100g d'eau oxygénée 30%. On laisse à cette température pendant 2 heures. On obtient ainsi 271g de N-oxyde brut. Ces 271g de N-oxyde sont engagés dans la réaction de transformation par addition de 320g d'eau, 168g d'acide acétique (2,8 moles); 1,88g (0,016 mole) de vanadate d'ammonium et 2000 ml d'hexane. Le mélange réactionnel est chauffé à 60°C pendant 30 minutes. Après refroidissement, la couche organique est séparée par décantation. Le citral est dosé par CPV avec étalonnage interne. On obtient 64,6g (0,42 mole) de citral, soit un rendement par rapport au myrcène de 42%.

Les rendements intermédiaires sont donnés dans le tableau suivant:

| | Nombre de moles | RR de l'étape | RR / myrcène |
|---|---|---|---|
| MYRCENE | 1 | - | - |
| DEGA | 0,79 | 79% | 79% |
| N-OXYDE | 0,68 | 86% | 68% |
| CITRAL | 0,42 | 62% | 42% |

RR = Rendement réel

DEGA = Diéthylgéranylamine

## Revendications

1. Procédé de préparation d'aldéhydes dans lequel on fait réagir un N-oxyde d'amine tertiaire dans un milieu aqueux en présence d'un catalyseur à base de vanadium caractérisé en ce que l'on opère en présence d'un solvent organique et d'une quantité catalytique d'un ou de plusieurs dérivés du vanadium.

2. Procédé selon la revendication 1 caractérisé en que le N-oxyde de l'amine tertiaire a pour formule générale :

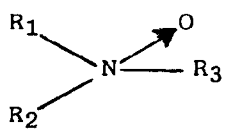

(I)

dans laquelle $R_1$, $R_2$ et $R_3$, égaux ou différents sont des groupements alkyl ou alkényl, linéaires, ramifiés ou cycliques, ou des groupements benzyl, lesquels groupements, substitués ou non, contiennent 1 à 30 atomes de carbone et éventuellement 1 ou plusieurs hétéroatomes, et dans laquelle $R_1$ et $R_2$ peuvent former entre eux un cycle.

3. Procédé selon la revendication 2 caractérisé en ce que $R_1$ et $R_2$, égaux ou différents, sont des groupements alkyl linéaires ou ramifiés, contenant 1 à 4 atomes de carbone, et pouvant former entre eux un cycle ou un hétérocycle.

4. Procédé selon la revendication 2 caractérisé en ce que $R_1$ et $R_2$, égaux ou différents, représentent un groupement méthyl ou éthyl.

5. Procédé selon la revendication 2 caractérisé en ce que $R_3$ est un groupement allylique ayant entre 3 et 30 atomes de carbone, linéaire ou ramifié, substitué ou non, contenant ou non un cycle et contenant éventuellement un ou plusieurs hétéroatomes.

6. Procédé selon la revendication 2 caractérisé en ce que $R_3$ est un groupement benzylique substitué ou non, contenant jusqu'à 30 atomes de carbone et éventuellement un ou plusieurs hétéroatomes.

7. Procédé selon la revendication 5 caractérisé en ce que $R_3$ est un groupement allylique contenant entre 3 et 15 atomes de carbone.

8. Procédé selon l'une des revendications précédentes caractérisé en ce que le N-oxyde d'amine est choisi parmi le N-oxyde de la N-dialkylprénylamine, le N-oxyde de la N-dialkylgéranylamine, le N-oxyde de la

N-dialkylbenzylamine et le N-oxyde de la N-dialkyl(triméthyl-2,6,6 cyclohéxène-1) méthanamine.

**9.** Procédé selon la revendication 8 caractérisé en ce que le N-oxyde d'amine est choisi parmi le N-oxyde de la N-diéthylgéranylamine, le N-oxyde de la N-diéthylprénylamine et le N-oxyde de la N-diéthyl(triméthyl-2,6,6 cyclohéxène-1) méthanamine.

**10.** Procédé selon la revendication 1 caractérisé en ce que le rapport molaire dérivés du vanadium (compté en métal)/N-oxyde est compris entre 0,0001 et 0,5 et de préférence entre 0,001 et 0,1.

**11.** Procédé selon la revendication 1 caractérisé en ce que le solvant est choisi parmi les hydrocarbures, les éthers, les esters, les alcools, les solvants aromatiques et les solvants halogénés.

**12.** Procédé selon la revendication 11 caractérisé en ce que le solvant est choisi parmi le pentane, l'hexane, l'acétate d'éthyle, le méthylterbutyléther, le chlorobenzène, le chlorure de méthylène, le toluène, le xylène, le benzène, et le méthanol.

**13.** Procédé selon la revendication 1 caractérisé en ce que le ou les dérivés du vanadium sont choisis parmi $V_2O_5$, $NH_4VO_3$, les vanadate d'alcoyle tels que l'orthovanadate de triéthanolamine (OVTEA) et l'orthovanadate d'octadécyle $OV(OC_{18}H_{37})_3$, l'acétylacétonate de vanadyle $OV(AcAc)_2$ ou le sulfate de vanadyle $OVSO_4$.

**14.** Procédé selon la revendication 1 caractérisé en ce que le pH du milieu réactionnel est ajusté par l'ajout d'un acide organique ou minéral ou des deux, et de préférence d'un acide carboxylique.

**15.** Procédé selon la revendication 1 caractérisé en ce que le milieu réactionnel contient, par mole de N-oxyde, entre 0,05 et 2 litres d'eau, et un volume de solvant inférieur à 20 litres, et de préférence compris entre 0,5 et 10 litres.

**16.** Procédé selon la revendication 1 caractérisé en ce que le pH du milieu est ajusté de préférence à une valeur comprise entre 4 et 7.

**17.** Procédé selon la revendication 1 caractérisé en ce que la température est comprise entre la température ambiante et 150°C.

**18.** Procédé selon la revendication 1 caractérisé en ce que, dans une première étape, on fait réagir le N-oxyde d'amine tertiaire sur le ou les dérivés du vanadium selon l'une des revendications 1 à 17, et, dans une deuxième étape, on récupère dans la phase organique l'aldéhyde et dans la phase aqueuse l'amine secondaire.

**19.** Procédé de préparation d'aldéhydes insaturés caractérisé en ce que l'on effectue, sans isoler les intermédiaires réactionnels, les étapes suivantes:

. dans une première étape, on condense un diène conjugué et une amine secondaire,
. dans une deuxième étape, on oxyde l'amine tertiaire ainsi obtenue au moyen d'un agent oxydant, et
. dans une troisième étape, on transforme le N-oxyde d'amine tertiaire obtenu en aldéhyde insaturé selon l'une des revendications 1 à 20.

**20.** Procédé selon la revendication 19 caractérisé en ce que le diène conjugué est choisi parmi les groupements alkényl linéaires, ramifiés, ou cycliques, substitués ou non, contenant 1 à 30 atomes de carbone et éventuellement 1 ou plusieurs hétéroatomes.

**21.** Procédé selon la revendication 20 caractérisé en ce que le diène conjugué est choisi parmi le myrcène, l'isoprène, le butadiène, le farnésène et le phytadiéne.

**22.** Procédé selon la revendication 19 caractérisé en ce que l'amine secondaire a pour formule HNR1R2 dans laquelle R1 et R2 sont définis comme dans la revendication 2.

**23.** Procédé selon la revendication 19 caractérisé en ce que, au cours de la première étape, on utilise un catalyseur choisi parmi les métaux alcalins.

**24.** Procédé selon la revendication 19 caractérisé en ce que le rapport molaire amine/diène est compris entre

0,1 et 5, et de préférence entre 0,5 et 2.

**25.** Procédé selon la revendication 19 caractérisé en ce que la première étape est conduite à une température comprise entre la température ambiante et la température de reflux.

**26.** Procédé selon la revendication 19 caractérisé en ce que, au cours de la deuxième étape, on utilise comme agent oxydant l'eau oxygénée.

## Claims

**1.** Process for preparing aldehydes, in which a tertiary amine N-oxide is reacted in an aqueous medium in the presence of a vanadium-based catalyst, characterized in that the reaction is performed in the presence of an organic solvent and of a catalytic quantity of one or more vanadium derivatives.

**2.** Process according to claim 1, characterized in that the tertiary amine N-oxide is of the general formula:

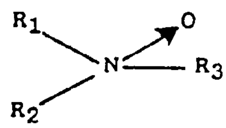

(I)

in which $R_1$, $R_2$ and $R_3$, which may be the same or different, are linear, branched or cyclic alkyl or alkenyl groups or benzyl groups, which groups, substituted or unsubstituted, contain 1 to 30 carbon atoms and optionally 1 or more hetero atoms, and in which formula $R_1$ and $R_2$ together can form a ring.

**3.** Process according to claim 2, characterized in that $R_1$ and $R_2$, which may be the same or different, are linear or branched alkyl groups containing 1 to 4 carbon atoms and which, together, can form a ring or a heterocycle.

**4.** Process according to claim 2, characterized in that $R_1$ and $R_2$, which may be the same or different, represent a methyl or ethyl group.

**5.** Process according to claim 2, characterized in that $R_3$ is a substituted or unsubstituted, linear or branched allyl group having between 3 and 30 carbon atoms, containing or not containing a ring and optionally containing one or more hetero atoms.

**6.** Process according to claim 2, characterized in that $R_3$ is a substituted or unsubstituted benzyl group containing up to 30 carbon atoms and optionally one or more hetero atoms.

**7.** Process according to claim 5, characterized in that $R_3$ is an allyl group containing between 3 and 15 carbon atoms.

**8.** Process according to one of the preceding claims, characterized in that the amine N-oxide is selected from N,N-dialkylprenylamine N-oxide, N,N-dialkylgeranylamine N-oxide, N,N-dialkyl-benzylamine N-oxide and N,N-dialkyl-(2,6,6-trimethyl-1-cyclohexenyl)-methanamine N-oxide.

**9.** Process according to claim 8, characterized in that the amine N-oxide is selected from N,N-diethylgeranylamine N-oxide, N,N-diethyl-prenylamine N-oxide and N,N-diethyl-(2,6,6-trimethyl-1-cyclohexenyl)-methanamine N-oxide.

**10.** Process according to claim 1, characterized in that the mole ratio vanadium derivatives (reckoned as metal)/N-oxide is between 0.0001 and 0.5, and preferably between 0.001 and 0.1.

**11.** Process according to claim 1, characterized in that the solvent is selected from hydrocarbons, ethers, esters, alcohols, aromatic solvents and halogenated solvents.

**12.** Process according to claim 11, characterized in that the solvent is selected from pentane, hexane, ethyl acetate, methyl tert-butyl ether, chlorobenzene, methylene chloride, toluene, xylene, benzene and methanol.

13. Process according to claim 1, characterized in that the vanadium derivative or derivatives are selected from $V_2O_5$, $NH_4VO_3$, alkyl vanadates such as triethanolamine orthovanadate (OVTEA) and octadecyl orthovanadate $OV(OC_{18}H_{37})_3$, vanadyl acetylacetonate $OV(AcAc)_2$ or vanadyl sulphate $OVSO_4$.

14. Process according to claim 1, characterized in that the pH of the reaction medium is adjusted by adding an organic or inorganic acid or both, and preferably a carboxylic acid.

15. Process according to claim 1, characterized in that the reaction medium contains, per mole of N-oxide, between 0.05 and 2 litres of water and a volume of solvent of less than 20 litres, and preferably between 0.5 and 10 litres.

16. Process according to claim 1, characterized in that the pH of the medium is preferably adjusted to a value between 4 and 7.

17. Process according to claim 1, characterized in that the temperature is between room temperature and 150°C.

18. Process according to claim 1, characterized in that, in a first step, the tertiary amine N-oxide is reacted with the vanadium derivative or derivatives according to one of claims 1 to 17, and in a second step, the aldehyde is recovered in the organic phase and the secondary amine in the aqueous phase.

19. Process for preparing unsaturated aldehydes, characterized in that the following steps are performed without isolating the reaction intermediates:
   . in a first step, a conjugated diene and a secondary amine are condensed,
   . in a second step, the tertiary amine thereby obtained is oxidized by means of an oxidizing agent, and
   . in a third step, the tertiary amine N-oxide obtained is converted to an unsaturated aldehyde according to one of claims 1 to 20.

20. Process according to claim 19, characterized in that the conjugated diene is selected from substituted or unsubstituted, linear, branched or cyclic alkenyl groups containing 1 to 30 carbon atoms and optionally 1 or more hetero atoms.

21. Process according to claim 20, characterized in that the conjugated diene is selected from myrcene, isoprene, butadiene, farnesene and phytadiene.

22. Process according to claim 19, characterized in that the secondary amine is of the formula $HNR_1R_2$ in which $R_1$ and $R_2$ are defined as in claim 2.

23. Process according to claim 19, characterized in that, during the first step, a catalyst selected from alkali metals is used.

24. Process according to claim 19, characterized in that the mole ratio amine/diene is between 0.1 and 5, and preferably between 0.5 and 2.

25. Process according to claim 19, characterized in that the first step is performed at a temperature between room temperature and the refluxing temperature.

26. Process according to claim 19, characterized in that, during the second step, hydrogen peroxide is used as an oxidizing agent.

**Patentansprüche**

1. Verfahren zur Herstellung von Aldehyden, worin man ein N-Oxid eines tertiären Amins in einem wäßrigen Milieu in Gegenwart eines Katalysators auf Basis von Vanadin umsetzt, dadurch gekennzeichnet, daß man in Gegenwart eines organischen Lösungsmittels und einer katalytischen Menge eines oder mehrerer Vanadinderivate arbeitet.

2. Verfahren gemäß Anspruch 1, dadurch gekennzeichnet, daß das N-Oxid des tertiären Amins die allgemeine Formel

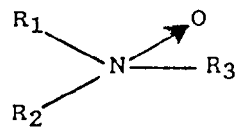

(I)

besitzt, worin $R_1$, $R_2$ und $R_3$, gleich oder verschieden, lineare, verzweigte oder cyclische Alkyl- oder Alkenylgruppen oder Benzylgruppen sind, wobei diese Gruppen, substituiert oder unsubstituiert, 1 bis 30 Kohlenstoffatome und gegebenenfalls 1 oder mehrere Heteroatome aufweisen, und worin $R_1$ und $R_2$ miteinander einen Ring bilden können.

3. Verfahren gemäß Anspruch 2, dadurch gekennzeichnet, daß $R_1$ und $R_2$, gleich oder verschieden, lineare oder verzweigte Alkylgruppen mit 1 bis 4 Kohlenstoffatomen sind und miteinander einen Ring oder einen Heterocyclus bilden können.

4. Verfahren gemäß Anspruch 2, dadurch gekennzeichnet, daß $R_1$ und $R_2$, gleich oder verschieden, einen Methyl- oder Ethylrest bedeuten.

5. Verfahren gemäß Anspruch 2, dadurch gekennzeichnet, daß $R_3$ eine lineare oder verzweigte, substituierte oder unsubstituierte allylische Gruppe mit zwischen 3 und 30 Kohlenstoffatomen ist, die gegebenenfalls einen Ring enthält und gegebenenfalls ein oder mehrere Heteroatome aufweist.

6. Verfahren gemäß Anspruch 2, dadurch gekennzeichnet, daß $R_3$ eine substituierte oder unsubstituierte Benzylgruppe ist, die bis zu 30 Kohlenstoffatome und gegebenenfalls ein oder mehrere Heteroatome aufweist.

7. Verfahren gemäß Anspruch 5, dadurch gekennzeichnet, daß $R_3$ eine allylische Gruppe mit zwischen 3 und 15 Kohlenstoffatomen ist.

8. Verfahren gemäß einem der vorhergehenden Ansprüche, dadurch gekennzeichnet, daß das N-Oxid des Amins unter dem N-Oxid von N-Dialkylprenylamin, dem N-Oxid von N-Dialkylgeranylamin, dem N-Oxid von N-Dialkylbenzylamin und dem N-Oxid von N-Dialkyl-(2,6,6-trimethyl-1-cyclohexen)-methanamin ausgewählt wird.

9. Verfahren gemäß Anspruch 8, dadurch gekennzeichnet, daß das N-Oxid des Amins unter dem N-Oxid von N-Diethylgeranylamin, dem N-Oxid von N-Diethylprenylamin und dem N-Oxid von N-Diethyl-(2,6,6-trimethyl-1-cyclohexen)-methanamin ausgewählt wird.

10. Verfahren gemäß Anspruch 1, dadurch gekennzeichnet, daß das Molverhältnis Vanadinderivate (gerechnet als Metall)/N-Oxid zwischen 0,0001 und 0,5 und vorzugsweise zwischen 0,001 und 0,1 liegt.

11. Verfahren gemäß Anspruch 1, dadurch gekennzeichnet, daß das Lösungsmittel unter Kohlenwasserstoffen, Ethern, Estern, Alkoholen, aromatischen Lösungsmitteln und halogenierten Lösungsmitteln ausgewählt wird.

12. Verfahren gemäß Anspruch 11, dadurch gekennzeichnet, daß das Lösungsmittel unter Pentan, Hexan, Ethylacetat, Methyl-tert.-butylether, Chlorbenzol, Methylenchlorid, Toluol, Xylol, Benzol und Methanol ausgewählt wird.

13. Verfahren gemäß Anspruch 1, dadurch gekennzeichnet, daß das oder die Vanadinderivate unter $V_2O_5$, $NH_4VO_3$, den Alkylvanadaten wie Triethanolamin-orthovanadat (OVTEA) und Octadecyl-orthovanadat $OV(OC_{18}H_{37})_3$,
Vanadylacetylacetonat $OV(AcAc)_2$ oder Vanadylsulfat $OVSO_4$ ausgewählt wird.

14. Verfahren gemäß Anspruch 1, dadurch gekennzeichnet, daß der pH des Reaktionsmilieus durch Zusatz einer organischen oder mineralischen Säure oder beider und vorzugsweise einer Carbonsäure eingestellt wird.

15. Verfahren gemäß Anspruch 1, dadurch gekennzeichnet, daß das Reaktionsmilieu je Mol N-Oxid zwischen 0,05 und 2 Liter Wasser und ein Lösungsmittelvolumen von geringer als 20 Liter und vorzugsweise zwischen 0,5 und 10 Liter enthält.

16. Verfahren gemäß Anspruch 1, dadurch gekennzeichnet, daß der pH des Milieus vorzugsweise auf einen Wert zwischen 4 und 7 eingestellt wird.

17. Verfahren gemäß Anspruch 1, dadurch gekennzeichnet, daß die Temperatur zwischen Raumtemperatur und 150°C liegt.

18. Verfahren gemäß Anspruch 1, dadurch gekennzeichnet, daß man in einer ersten Stufe das N-Oxid des tertiären Amins mit dem oder den Vanadinderivaten gemäß einem der Ansprüche 1 bis 17 umsetzt und dann in einer zweiten Stufe in der organischen Phase den Aldehyd und in der wäßrigen Phase das sekundäre Amin wiedergewinnt.

19. Verfahren zur Herstellung von ungesättigten Aldehyden, dadurch gekennzeichnet, daß man ohne Isolierung der Reaktionszwischenprodukte die folgenden Stufen ausführt:
- man in einer ersten Stufe ein konjugiertes Dien und ein sekundäres Amin kondensiert,
- man in einer zweiten Stufe das so erhaltene tertiäre Amin mit einem Oxidationsmittel oxidiert und
- man in einer dritten Stufe das erhaltene N-Oxid des tertiären Amins in einen ungesättigten Aldehyd gemäß einem der Ansprüche 1 bis 18 überführt.

20. Verfahren gemäß Anspruch 19, dadurch gekennzeichnet, daß das konjugierte Dien unter den substituierten oder unsubstituierten, linearen, verzweigten oder cyclischen Alkenylgruppen, die 1 bis 30 Kohlenstoffatome und gegebenenfalls 1 oder mehrere Heteroatome aufweisen, ausgewählt wird.

21. Verfahren gemäß Anspruch 20, dadurch gekennzeichnet, daß das konjugierte Dien unter Myrcen, Isopren, Butadien, Farnesen und Phytadien ausgewählt wird.

22. Verfahren gemäß Anspruch 19, dadurch gekennzeichnet, daß das sekundäre Amin die Formel $HNR_1R_2$ aufweist, worin $R_1$ und $R_2$ wie in Anspruch 2 definiert sind.

23. Verfahren gemäß Anspruch 19, dadurch gekennzeichnet, daß man im Verlauf der ersten Stufe einen Katalysator, ausgewählt unter den Alkalimetallen, verwendet.

24. Verfahren gemäß Anspruch 19, dadurch gekennzeichnet, daß das Molverhältnis Amin/Dien zwischen 0,1 und 5 und vorzugsweise zwischen 0,5 und 2 liegt.

25. Verfahren gemäß Anspruch 19, dadurch gekennzeichnet, daß die erste Stufe bei einer Temperatur zwischen Raumtemperatur und Rückflußtemperatur durchgeführt wird.

26. Verfahren gemäß Anspruch 19, dadurch gekennzeichnet, daß man im Verlauf der zweiten Stufe als Oxidationsmittel Wasserstoffperoxid verwendet.